(19) Europäisches Patentamt
European Patent Office
Office européen des brevets

(11) Veröffentlichungsnummer: **0 355 663**
**A2**

# (12) EUROPÄISCHE PATENTANMELDUNG

(21) Anmeldenummer: **89115025.2**

(22) Anmeldetag: **15.08.89**

(51) Int. Cl.⁴: **C07D 487/04 , C08K 5/34 , //(C07D487/04,209:00,209:00)**

(30) Priorität: **16.08.88 DE 3827641**

(43) Veröffentlichungstag der Anmeldung: **28.02.90 Patentblatt 90/09**

(84) Benannte Vertragsstaaten: **BE CH DE ES FR GB IT LI NL**

(71) Anmelder: **BASF Aktiengesellschaft**
**Carl-Bosch-Strasse 38**
**D-6700 Ludwigshafen(DE)**

(72) Erfinder: **Aumueller, Alexander, Dr.**
**An der Marlach 5**
**D-6705 Deidesheim(DE)**
Erfinder: **Neumann, Peter, Dr.**
**Poststrasse 28**
**D-6800 Mannheim 31(DE)**
Erfinder: **Trauth, Hubert**
**Milanstrasse 5**
**D-6724 Dudenhofen(DE)**

## (54) 2,6-Polyalkylpiperidinsubstituierte Bislactame und deren Verwendung zum Stabilisieren von organischem Material, insbesondere von Kunststoffen und damit stabilisiertes Material.

(57) Bislactame der allgemeinen Formel I

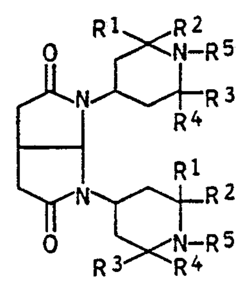

(I),

worin
$R^1$, $R^2$, $R^3$ und $R^4$ unabhängig voneinander $C_1$-$C_4$-Alkyl oder $R^1$ und $R^2$ und/oder $R^3$ und $R^4$ einen Tetra- oder Pentamethylenrest und
$R^5$ Wasserstoff, Carbonyl-$C_1$-$C_8$-acyl, $C_1$-$C_8$-Alkyl, $C_7$-$C_{10}$-Phenylalkyl, wobei der Phenylkern unsubstituiert oder durch ein oder zwei $C_1$-$C_4$-Alkyl, $C_1$-$C_4$-Alkoxy, Fluor, Chlor oder Brom substituiert ist, $C_1$-$C_3$-Cyanalkyl, $C_2$-$C_4$-Hydroxyalkyl oder $C_2$-$C_3$-Aminoalkyl bedeuten,
sowie die Säureadditionssalze und Hydrate dieser Verbindungen.
Die Verbindungen (I) sind Stabilisatoren für organische Materialien.

EP 0 355 663 A2

**2,6-Polyalkylpiperidinsubstituierte Bislactame und deren Verwendung zum Stabilisieren von organischem Material, insbesondere von Kunststoffen und damit stabilisiertes Material**

Es ist bekannt, daß Polyalkylpiperidinderivate organische Polymere vor Zerstörung durch Licht und Wärme schützen.

Unbefriedigend ist bei den Verbindungen des Standes der Technik häufig die geringe Verträglichkeit mit Polyolefinen und/oder anderen Kunststoffen, die Dauer der Schutzwirkung, die Eigenfarbe der Substanzen, die Neigung zur Flüchigkeit und die thermische Zersetzung der Stabilisatoren beim Einarbeiten in das Polymere bei erhöhter Temperatur.

Aufgabe der Erfindung war es, neue Stabilisatoren zur Verfügung zu stellen, welche die vorstehenden Nachteile nicht aufweisen.

Die Aufgabe wird mit Hilfe der Bislactame der Erfindung gelöst. Dementsprechend betrifft die Erfindung Bislactame der allgemeinen Formel I

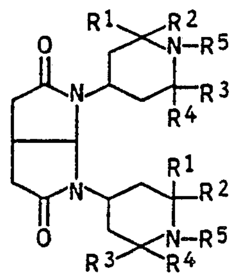

(I),

worin

$R^1$, $R^2$, $R^3$ und $R^4$ unabhängig voneinander $C_1$-$C_4$-Alkyl oder $R^1$ und $R^2$ und/oder $R^3$ und $R^4$ zusammen ein Tetra- oder Pentamethylenrest und

Wasserstoff, Carbonyl-$C_1$-$C_8$-alkyl, Benzoyl, , $C_1$-$C_8$-Alkyl, $C_7$-$C_{10}$-Phenylalkyl, wobei der Phenylkern unsubstituiert oder durch ein oder zwei $C_1$-$C_4$-Alkyl, $C_1$-$C_4$-Alkoxy, Fluor, Chlor oder Brom substituiert ist, $C_1$-$C_3$-Cyanalkyl, $C_2$-$C_4$-Hydroxyalkyl oder $C_2$-$C_3$-Aminoalkyl bedeuten,

sowie die Säureadditionssalze und Hydrate dieser Verbindungen.

Die Verbindungen (I) sind wegen ihrer guten Verträglichkeit mit organischen Materialien, vor allem in Kunststoffen und Lackbindemitteln hervorragend als Stabilisatoren für diese Mittel geeignet. Die farblosen Verbindungen sind nicht flüchtig und so stabil, daß diese bei erhöhten Temperaturen in Kunststoffe eingearbeitet werden können.

Die erfindungsgemäßen Verbindungen sind vor allem zur Stabilisierung von Polyolefinen, insbesondere von Ethylen- und Propylenpolymerisaten, von Polyurethanen und Lacken geeignet.

Alkylgruppen für $R^1$, $R^2$, $R^3$ und $R^4$ sind beispielsweise Methyl, Ethyl, n-Propyl, iso-Propyl, n-Butyl oder iso-Butyl. Zwei benachbarte Reste $R^1$ und $R^2$ und/oder $R^3$ und $R^4$ können zusammen auch ein Tetra- oder Pentamethylenrest sein.

Vorzugsweise stehen $R^1$, $R^2$, $R^3$ und $R^4$ für Methyl.

Alkylreste für $R^5$ können linear oder verzweigt sein. Im einzelnen seien beispielsweise Methyl, Ethyl, Propyl, n-Butyl, n-Pentyl, iso-Pentyl, n-Hexyl, n-Heptyl oder n-Octyl genannt. Bevorzugter Alkylrest für $R^5$ ist Methyl.

Carbonylalkylreste für $R^5$ sind beispielsweise Propionyl, Butanoyl, Pentanoyl, Hexanoyl, Heptanoyl, Octanoyl, außerdem Benzoyl und insbesondere Acetyl. Phenylalkylreste für $R^5$ sind beispielsweise Phenylethyl und vorzugsweise Benzyl.

Als am Phenylkern substituierte Phenylalkylreste $R^5$ sind z.B. zu nennen: 3- und 4-Methoxybenzyl, 3- und 4-Methoxyphenylethyl, 3- und 4-Chlorbenzyl, 3-oder 4-Chlorphenylethyl, 3- und 4-Ethoxybenzyl, 3- und 4-Ethoxyphenylethyl und 3- und 4-Methylbenzyl, von denen 3- und 4-Methylbenzyl bevorzugt sind. Cyanalkylreste für $R^5$ sind beispielsweise Cyanethyl, vorzugsweise Cyanmethyl.

Als Hydroxyalkyl und Aminoalkyl kommen für $R^5$ z.B. in Betracht: 3-Hydroxypropyl, 4-Hydroxybutyl, 2-Hydroxyethyl, 3-Aminopropyl, 2-Aminoethyl. Von diesen sind 2-Hydroxyethyl und 2-Aminoethyl bevorzugt. Besonders bevorzugt für $R^5$ ist Wasserstoff.

Die Verbindungen (I) lassen sich analog der DE-A 2 325 429 durch Aminolyse der Verbindung der Formel (II) mit Aminen der allgemeinen Formel (III) mit oder ohne Lösungsmittel bzw. mit oder ohne

Katalysator herstellen. Vorzugsweise arbeitet man mit einem Überschuß an Amin ohne Lösungsmittel und Katalysator bei Temperaturen zwischen 100 und 220 °C, bevorzugt bei 120 bis 150 °C.

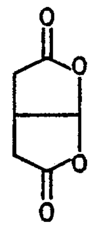

(II)

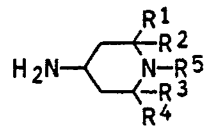

(III)

Verbindungen der allgemeinen Formel (I) mit $R^5$ gleich Wasserstoff lassen sich durch an sich literaturbekannte Verfahren wie Alkylierung, Acylierung, Michaeladdition, Cyanmethylierung oder Hydroxyalkylierung in Verbindungen der allgemeinen Formel (I) mit $R^5$ ungleich Wasserstoff umwandeln.

Die erfindungsgemäßen Verbindungen (I) können in Form der freien Basen, als Hydrate oder als Salze vorliegen. Geeignete Anionen stammen z. B. von anorganischen Säuren, Carbonsäuren und Sulfonsäuren. Von den Salzen sind die der Carbonsäuren und Sulfonsäuren bevorzugt.

Als anorganische Anionen kommen z.B. Chlorid, Bromid, Sulfat, Methosulfat, Tetrafluoroborat, Phosphat und Rhodanid in Betracht.

Carbonsäure-Anionen sind beispielsweise: Formiat, Acetat, Propionat, Hexanoat, Cyclohexanoat, Lactat, Stearat, Palmitat, Dodecylbenzoat, Benzoat, Acrylat, Methacrylat, Zitrat, Malonat oder Succinat sowie Anionen von Polycarbonsäuren, wie Polyacrylsäure, Polymethacrylsäure und von (Meth)-Acrylsäure enthaltenden Copolymeren mit bis zu 3000 COOH-Gruppen.

Sulfonsäure-Anionen sind beispielsweise Benzolsulfonat, Tosylat und Methylsulfonat.

Den zu stabilisierenden Kunststoffen werden die Verbindungen (I) in einer Konzentration von 0,01 bis 5 Gew.-% vorzugsweise 0,02 bis 2 Gew.-% vor, während oder nach der Polymerbildung zugesetzt.

Zur Herstellung der Gemische aus den erfindungsgemäßen Verbindungen und den zu stabilisierenden Kunststoffen können alle bekannten Verfahren zum Einmischen von Stabilisierungsmitteln oder anderen Zusätzen in Polymere angewandt werden.

Neben den erfindungsgemäßen Verbindungen (I) können die stabilisierten Kunststoffe gegebenenfalls noch weitere Additive enthalten, beispielsweise Antioxidantien, zusätzliche Lichtstabilisierungsmittel, Metalldesaktivatoren, antistatische Mittel, flammhemmende Mittel, außerdem Pigmente und Füllstoffe.

Antioxidantien und Lichtstabilisatoren, die den Kunststoffen neben den erfindungsgemäßen Verbindungen zugesetzt werden können, sind z. B. Verbindungen auf der Basis sterisch gehinderter Phenole, Schwefel und/oder Phosphor enthaltende Costabilisatoren.

Als derartige phenolische Antioxidationsmittel seien beispielswiese 2,6-Di-tert.-butyl-4-methylphenol, n-Octadecyl-β-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isoOcyanurat, 1,3,5-Tris-[β-(3,5-di-tert.-butyl-4-hydroxyphenyl)-propionyloxyethyl]-isocyanurat, 1,3,5-Tris-(2,6,di-methyl-3-hydroxy-4-tert.-butyl-benzyl)-isocyanurat und Pentaerythrit-tetrakis-[β-(3,5,di-tert.-butyl-4-hydroxy-phenyl)-propionat] erwähnt.

Als phosphorhaltige Antioxidantien kommen z.B. Tris-(nonyl-phenyl)-phosphit, Distearylpentaerythritdiphosphit, Tris-(2,4-di-tert.-butylphenyl)-phosphit, Tris-(2-tert.-butyl-4-methylphenyl)-phosphit, Bis-(2,4-ditert.-butylphenyl)-pentaerythritdiphosphit und Tetrakis-(2,4-di-tert.-butylphenyl)-4,4′-biphenylendiphosphit in Betracht.

Als Schwefel enthaltende Antioxidationsmittel seien beispielsweise Dilaurylthiodipropionat, Dimyristylthiodipropionat, Distearylthiodipropionat, Pentaerythrittetrakis-(β-laurylthiopropionat) und Pentaerythrittetrakis-(β-hexylthiopropionat) erwähnt.

Weitere Antioxidantien und Lichtstabilisatoren, die zusammen mit den erfindungsgemäßen Verbindungen verwendet werden können, sind z. B. 2-(2′-Hydroxyphenyl)-benztriazole, 2-Hydroxybenzophenone, Phenylester von Hydroxybenzoesäuren, α-Cyanozimtsäurederivate, Nickelverbindungen, Benzimidazolcarbonsäureanilide und/oder Oxalsäuredianilide.

Als organische polymere, die durch die erfindungsgemäßen Verbindungen stabilisiert werden können, kommen z. B. in Betracht:

Polymer von Mono- und Diolefinen, wie Polyethylen niedriger und hoher Dichte, lineares Polyethylen niedriger Dichte, Polypropylen, Polyiso butylen, Polybuten-1, Polyisopren, Polybutadien sowie Copolymerisate von Mono- oder Diolefinen und Mischungen der genannten Polymeren;
Copolymerisate von Mono- oder Diolefinen mit anderen Vinylmonomeren z. B. Ethylen-Alkylacrylat-Copolymere, Ethylen-Alkylmethacrylat-Copolymere, Ethylen-Vinylacetat-Copolymere oder Ethylen-Acrylsäure-Copolymere;
Polystyrol;
Copolymere von Styrol oder α-Methylstyrol mit Dienen oder Acrylderivaten, wie Styrol-Butadien, Styrol-Acrylnitril, Styrol-Ethylmethacrylat, Styrol-Butadien-Ethylacrylat, Styrol-Acrylnitril-Methacrylat;
ABS-, MBS- oder ähnliche Polymere;
Halogenhaltige Polymere, z. B. Polyvinylchlorid, Polyvinylfluorid, Polyvinylidenfluorid sowie deren Copolymere;
Polymere die sich von α,β-ungesättigten Säuren und deren Derivaten ableiten, wie Polyacrylate und Polymethacrylate, Polyacrylamide und Polyacrylnitrile;
Polymere, die sich von ungesättigten Alkoholen und Aminen bzw. deren Acrylderivaten oder Acetalen ableiten, wie Polyvinylalkohol oder Polyvinylacetat;
Polyurethane, Polyamide, Polyharnstoffe, Polyester, Polycarbonate, Polysulfone, Polyethersulfone und Polyetherketone.

Weitere organische Polymere, die mit den erfindungsgemäßen Verbindungen stabilisiert werden können, sind Lacküberzüge, von denen die Einbrennlackierungen, von diesen wiederum Fahrzeuglackierungen, vorzugsweise Zweischichtlackierungen, besonders hervorzuheben sind. Auch hier können die bereits genannten weiteren Antioxidantien und Lichtschutzmittel noch zugesetzt werden.

Die erfindungsgemäßen Verbindungen können dem Lack in fester oder gelöster Form zugegeben werden. Die gute Löslichkeit von (I) in den Lacksystemen ist dabei von besonderem Vorteil.

Beispiel

10 g 2,8-Dioxa-cis-bicyclo[3.3.0]octa-3,7-dion (Herstellung: Tetrahedron 36, 321 (1980)) wurden in 80 ml 4-Amino-2,2,6,6-tetramethylpiperidin 3 h auf 120 °C und anschließend 5,5 h auf 150 °C erhitzt. Nach dem Abkühlen wurden 250 ml Petrolether zugegeben, der ausgefallene Niederschlag wurde abgesaugt, mit Petrolether gewaschen, mit Ethanol ausgekocht und heiß abgesaugt. Man erhielt 24,0 g der Verbindung der Formel

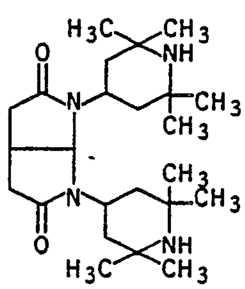

als farblosen Feststoff vom Schmp. 257 °C. Die Verbindung kristallisierte mit einem Mol Kristallwasser.

| | | | | |
|---|---|---|---|---|
| Ber.: | C 66,0 | H 10,2 | N 12,8 | O 11,0 |
| Gef.: | C 65,9 | H 10,1 | N 12,6 | O 11,4 |

## Ansprüche

1. Bislactame der allgemeinen Formel I

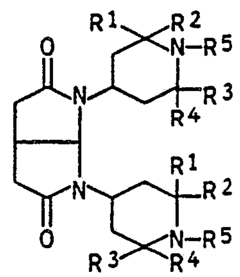

(I),

worin

$R^1$, $R^2$, $R^3$ und $R^4$ unabhängig voneinander $C_1$-$C_4$-Alkyl oder $R^1$ und $R^2$ und/oder $R^3$ und $R^4$ zusammen ein Tetra- oder Pentamethylenrest und

$R^5$ Wasserstoff, Carbonyl-$C_1$-$C_8$-alkyl, Benzoyl, $C_1$-$C_8$-Alkyl, $C_7$-$C_{10}$-Phenylalkyl, wobei der Phenylkern unsubstituiert oder durch ein oder zwei $C_1$-$C_4$-Alkyl, $C_1$-$C_4$-Alkoxy, Fluor, Chlor oder Brom substituiert ist, $C_1$-$C_3$-Cyanalkyl, $C_2$-$C_4$-Hydroxyalkyl oder $C_2$-$C_3$-Aminoalkyl bedeuten,

sowie die Säureadditionssalze und Hydrate dieser Verbindungen.

2. Verbindungen gemäß Anspruch 1, dadurch gekennzeichnet, daß $R^1$, $R^2$, $R^3$ und $R^4$ Methyl bedeuten.

3. Verbindungen gemäß Anspruch 1 oder 2, dadurch gekennzeichnet, daß $R^5$ Wasserstoff bedeutet.

4. Verwendung von Verbindungen gemäß Anspruch 1, 2 oder 3 zum Stabilisieren von organischem Material.

5. Verwendung von Verbindungen gemäß Anspruch 1, 2 oder 3 zum Stabilisieren von Kunststoffen.

6. Verwendung von Verbindungen gemäß Anspruch 1, 2 oder 3 zum Stabilisieren von Polyolefinen, Polyurethanen, ABS oder Polyamiden.

7. Verwendung von Verbindungen gemäß Anspruch 1, 2 oder 3 zum Stabilisieren von Lacken.

8. Verwendung von Verbindungen gemäß Anspruch 1, 2 oder 3 als Metallionendesaktivator.

9. Stabilisiertes organisches Material, enthaltend mindestens eine Verbindung gemäß Anspruch 1, 2 oder 3.